# EUROPEAN PATENT APPLICATION

(11) **EP 3 673 819 A1**
(43) Date of publication of application: **01.07.2020**
(21) Application number: 18848192.3
(22) Date of filing: 09.07.2018
(51) Int. Cl.: A61B 17/072, A61B 17/32

(54) **REUSABLE ELECTRIC ENDOSCOPE CUTTER STAPLER**

(30) Priority: 25.08.2017 CN 201710743398
(71) Applicant: Shanghai Yisi Medical Technology Co., Ltd., Shanghai 201318 (CN); Yisi Suzhou Medical Technology Co., Ltd., Suzhou City, Jiangsu 215163 (CN)
(72) Inventor: WANG, Jianbing, Shanghai 201318 (CN); NIE, Honglin, Shanghai 201318 (CN); CHEN, Jidong, Shanghai 201318 (CN); DING, Hao, Shanghai 201318 (CN); TAO, Yingying, Shanghai 201318 (CN)
(74) Representative: Strachan, Victoria Jane
(86) International application number: PCT/CN2018/094986
(87) International publication number: WO 2019/037552

(57) **Abstract**

The present application provides a reusable powered endoscopic cutter stapler, comprising a controller, a handle, an adapter and a cable component; the adapter is connected with the handle; one end of the cable component is detachably connected with the controller, the other end is detachably connected with the handle to be used for transmitting electric energy and command signals; the controller can be reused for many times, and the cable component can be reused for many times after being sterilized. According to the reusable powered endoscopic cutter stapler disclosed by the present application, at least one or multiple components of a core component can be sterilized repeatedly, and can be used for many times, thereby not only greatly lowering the treatment expense of patients, but also greatly reducing the risk of cross infection when instruments are used, improving the use safety and reliability of the instruments, and greatly lightening the overall mass, so that a doctor can operate more conveniently.

## Description

### TECHNICAL FIELD

The present application relates to a surgical operation instrument, and particularly relates to a reusable powered endoscopic cutter stapler, used for transection, resection, and anastomoses of lung, stomach and intestinal tissues. The instruments have applications in multiple open or minimally invasive surgical procedures.

### BACKGROUD

Along with popularization of minimally invasive surgery, endoscopic cutter staplers have become a kind of conventional surgical instruments. Existing mechanical endoscopic cutter staplers are operated by hand, and have the deficiencies of being unstable in cutting, weak in firing force, low in reliability, strenuous in operation and the like, may cause unnecessary pain or trauma to patients in a treatment process, and also will bring hidden danger in health to the hand joints of doctors using the endoscopic cutter staplers for a long time. In recent years, disposable powered endoscopic cutter staplers appearing on the market may solve the above-mentioned problems, but the disposable powered endoscopic cutter staplers often bring economical burden for patients due to high cost and high price.

Currently, some reusable powered endoscopic cutter staplers also appear on the market which reduce the cost of patient in treatment by reusing instruments, however, core components of the staplers are not suitable for being sterilized repeatedly, and are only isolated from patients by a physical device in use and simply wiped after being used, to avoid cross infection. Therefore, when being treated by using instruments, great risk of cross infection is caused to the patient. Moreover, due to a motor and a battery pack inside, such kind of powered endoscopic cutter stapler is heavy in overall mass and brings inconvenience for doctors in operation.

### SUMMARY

In order to solve the foregoing technical problems, the present application provides an powered endoscopic cutter stapler which can be sterilized repeatedly, the stapler can be reused for many times after being sterilized, and thus solves the technical problem that powered staplers existing in the prior art cannot be sterilized repeatedly, and by unique product design, the overall mass of the stapler is lightened, and convenience for operation by doctors is improved.

According to an aspect of the present application, a reusable powered endoscopic cutter stapler is provided, which is used for transection, resection, and anastomoses of lung, stomach and intestinal tissues in multiple op en or minimally invasive surgical procedures, and can be sterilized repeatedly and reused for many times. The powered stapler which can be sterilized repeatedly includes: a controller, a handle, an adapter and a cable component; the adapter being connected with the handle; one end of the cable component can be detachably connected with the controller, the other end being detachably connected with the handle to be used for transmitting electric energy and command signals; the controller can be reused for many times, and the cable component can be reused for many times after being sterilized.

In a first implementation mode, the adapter is detachably connected with the handle; the adapter being a disposable component, and the handle can be reused for many times after being sterilized.

In a second implementation mode, the adapter and the handle combine a whole device, the whole device being reused for many times after being sterilized.

In a third implementation mode, the adapter and the handle combine a whole device, the whole device being a disposable component.

In a fourth implementation mode, the controller includes a battery pack and a control module which are detachably connected, electric energy of the controller can be supplied by the battery pack, the battery pack and the adapter being disposable components, and the handle can be reused for many times after being sterilized.

According to another aspect of the present application, a reusable powered endoscopic cutter stapler is provided, which is used for transection, resection, and anastomoses of lung, stomach and intestinal tissues in multiple open or minimally invasive surgical procedures, and can be sterilized repeatedly and used for many times. The powered stapler which can be sterilized repeatedly includes a controller, a handle and an adapter; the adapter being connected with the handle; the controller can be directly mounted on the handle and detachably connected with the handle, the controller including a battery pack and a control module, electric energy of the controller can be supplied by the battery pack, the adapter and the battery pack being disposable components, and the handle can be reused for many times after being sterilized.

Further, the controller includes a battery pack and a control module, the battery pack converting power frequency voltage into DC voltage needed by the handle; the control module including a circuit board component and a software program, and being in charge of the mechanism operation and indication of the handle.

Further, the handle acquires electric energy from the controller and operates and indicates the adapter according to the instruction of the controller, including firing or withdrawing a blade, and indicating the location and direction as well as residual use times of a handle.

Further, the handle includes a handle shell, a firing handle, a safety trigger, a return button, a stroke indicator light, a residual use time indicator light and a direction indicator light.

Further, the adapter includes an outer tube, a rotation collar and a rotation knob; the far end of the outer tube being loaded with a proper cartridge in use to be used for anastomosis.

According to the reusable powered endoscopic cutter stapler disclosed by the present application, at least one or multiple components of a core component can be sterilized repeatedly, and can be used for many times, thereby not only greatly lowering the treatment expense of patients, but also greatly reducing the risk of cross infection when instruments are used, by setting a component for contacting human tissues to a working terminal as a disposable component, or independently sterilizing the component at the working terminal, improving the use safety and reliability of the instruments, and greatly lightening the overall mass, so that a doctor can operate more conveniently. Moreover, a detachable battery pack is used for supplying energy for the controller, so that the stapler is not limited by use occasions, and portability is improved.

### BRIEF DESCRIPTION OF THE DRAWINGS

FIG. 1 is a schematic structure diagram of a reusable powered endoscopic cutter stapler according to implementation mode I of the present application;
FIG. 2 is a schematic structure diagram of a handle of the reusable powered endoscopic cutter stapler of FIG. 1;
FIG. 3 is a schematic structure diagram of an adapter of the reusable powered endoscopic cutter stapler of FIG. 1;
FIG. 4 is a schematic structure diagram of a reusable powered endoscopic cutter stapler according to implementation mode II and implementation mode III of the present application;
FIG. 5 is a schematic structure diagram of a reusable powered endoscopic cutter stapler according to implementation mode IV of the present application;
FIG. 6 is a schematic structure diagram of a reusable powered endoscopic cutter stapler according to implementation mode V of the present application.

### DESCRIPTION OF THE EMBODIMENTS

The following clearly and completely describes the technical schemes in the embodiments of the present invention. Apparently, the described embodiments are some of the embodiments of the present invention rather than all of the embodiments. All other embodiments obtained by a person of ordinary skill in the art based on the embodiments of the present invention without creative efforts shall fall within the protection scope of the present disclosure.

For the convenience of description, "near end" in the whole application refers to an end close to an operator when the operator holds an instrument, and "far end" refers to an end far away from an operator when the operator holds the instrument.

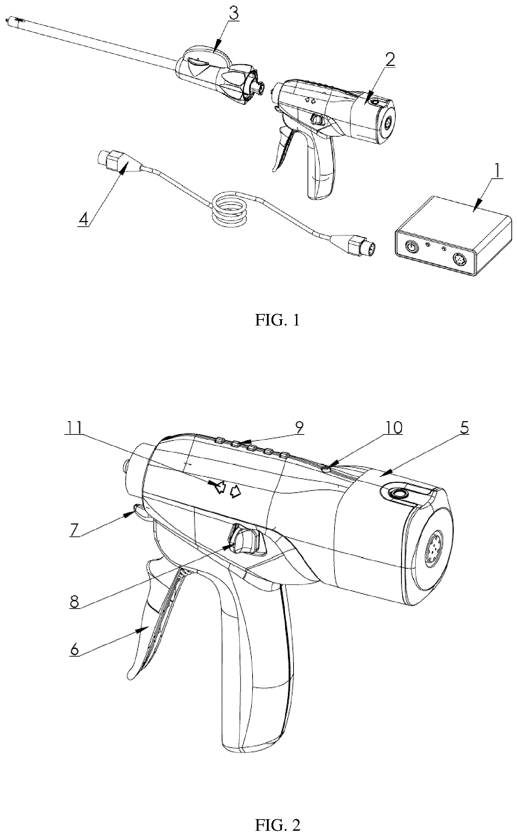

Referring to FIG. 1, a schematic structure diagram of a reusable powered endoscopic cutter stapler according to implementation mode I of the present application is shown. The reusable powered endoscopic cutter stapler is used for transection, resection, and anastomoses of lung, stomach and intestinal tissues in multiple open or minimally invasive surgical procedures, and includes a controller 1, a handle 2, an adapter 3 and a cable component 4; the adapter 3 being connected with the handle 2; one end of the cable component 4 being detachably connected with the controller 1, the other end being detachably connected with the handle 2 to be used for transmitting electric energy and command signals. The controller 1 can be reused for many times, the adapter 3 is a disposable component, and the handle 2 and the cable component 4 can be reused for many times after being sterilized. 50Hz, 220V power frequency voltage is accessed by the controller 1, the controller 1 includes a battery pack and a control module, the battery pack converts 220V power frequency voltage into 12V DC voltage needed by the handle 2; and the control module includes a power circuit component and a software program, and is in charge of the mechanism operation and indication of the handle 2.

Referring to FIG. 2, a schematic structure diagram of a handle of a reusable powered endoscopic cutter stapler in FIG. 1 is shown. A handle is an important operating and executing component of a stapler, is connected with the controller 1 by a cable component 4, acquires electric energy from the controller 1 and firing or withdraws a blade, and indicates the location and direction as well as residual use times of a handle. The handle 2 includes a handle case 5, a firing handle 6, a safety trigger 7, a return button 8, a stroke indicator light 9, a residual use time indicator light 10 and a direction indicator light 11. The adapter 3 is mounted on the handle 2 in use, and a far end of an outer tube 12 of the adapter 3 can be loaded with a proper cartridge to be used for anastomosis.

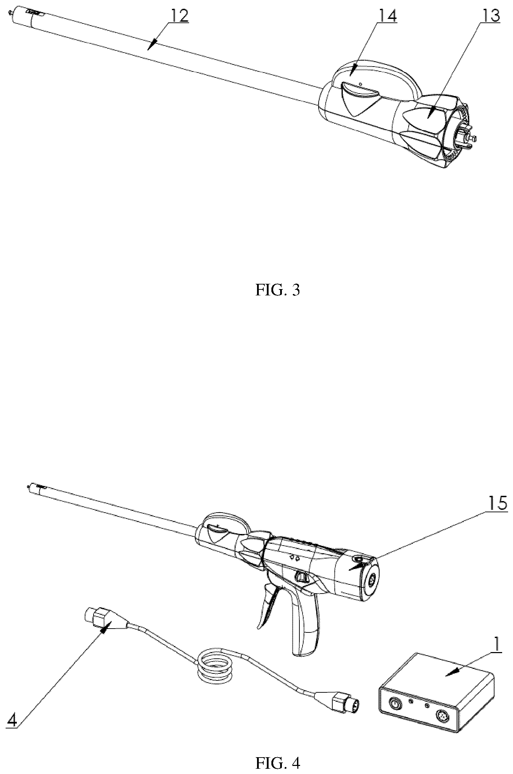

Referring to FIG. 3, a schematic structure diagram of an adapter of a reusable powered endoscopic cutter stapler in FIG. 1 is shown. The adapter 3 is mounted on the handle 2 in use, and a far end of the adapter can be loaded with a proper cartridge. The adapter 3 includes an outer tube 12, a rotation collar 13 and a rotation knob 14. The adapter is in aseptic packaging, is mounted on a handle 2 in use as being disposable, and is dismounted from the handle 2 after completing a surgery to be subject to waste treatment.

Before use of a reusable powered endoscopic cutter stapler according to implementation mode I, a controller 1 is in non-aseptic packaging, and a handle 2, an adapter 3 and a cable component are in aseptic packaging. In use, firstly, the controller 1 is connected with the handle 2 by a cable component 4, then the adapter 3 is mounted on the handle 2, and finally a proper cartridge is loaded an outer tube of the adapter 3 to perform the operation of anastomosis. After operation is ended, the controller 1 is stored according to specified storage requirements, the adapter 3 is subject to waste treatment as a disposable component, and the handle 2 and the cable component 4 are sterilized according to operation specifications, and are stored according to storage requirements for use next time.

### Implementation mode II:

The implementation mode is basically the same as the implementation mode I, and the only difference is that the handle 2 and the adapter 3 are made into a whole device 15, referring to FIG. 4, the whole device 15 can be reused for many times after being sterilized.

Before use of a reusable powered endoscopic cutter stapler according to implementation mode II, a controller 1 is in non-aseptic packaging, and a whole device 15 and a cable component 4 are in aseptic packaging. In use, firstly, the controller 1 is connected with a handle portion in the whole device 15 by a cable component 4, and then a proper cartridge is loaded in an outer tube of an adapter in the whole device 15 to perform the operation of anastomosis. After operation is ended, the controller 1 is stored according to specified storage requirements, the adapter 3 is subject to waste treatment as a disposable component, and the whole device 15 and the cable component 4 are sterilized according to operation specifications, and are stored according to storage requirements for use next time.

### Implementation mode III:

Further referring to FIG. 4, the implementation mode is basically the same as the implementation mode II, and the only difference is that the whole device 15 is disposable.

Before use of a reusable powered endoscopic cutter stapler according to implementation mode III, a controller 1 is in non-aseptic packaging, and a whole device 15 and a cable component 4 are in aseptic packaging. In use, firstly, the controller 1 is connected with a handle portion in the whole device 15 by a cable component 4, and then a proper cartridge is loaded in an outer tube of an adapter in the whole device 15 to perform the operation of anastomosis. After operation is ended, the controller 1 is stored according to specified storage requirements, the whole device 15 is subject to waste treatment as a disposable component, and the cable component 4 is sterilized according to operation specifications, and is stored according to storage requirements for use next time.

### Implementation mode IV:

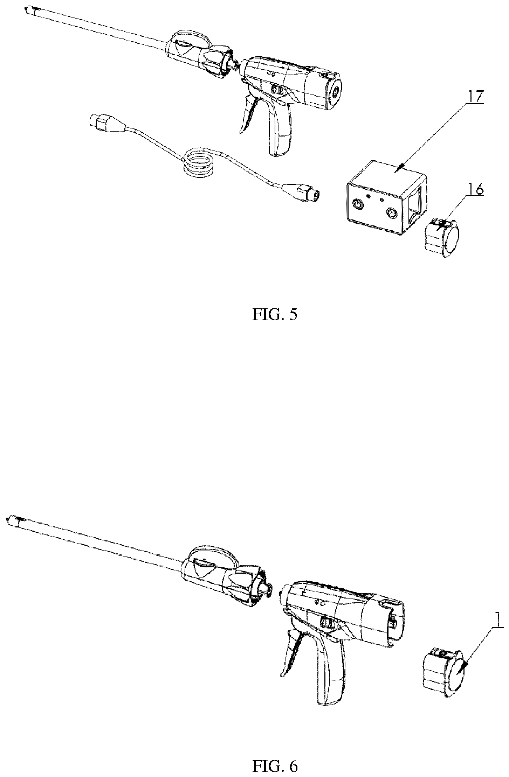

The implementation mode is basically the same as the implementation mode I, and the only difference is that a controller 1 includes a battery pack 16 and a control module 17 which are detachably connected, referring to FIG. 5. Electric energy of the controller 1 is supplied by the battery pack 16, and the battery pack 16 and the adapter 3 are disposable components.

Before use of a reusable powered endoscopic cutter stapler according to implementation mode IV, a battery pack 16 and a control module 17 are in non-aseptic packaging, and a handle 2, an adapter 3 and a cable component 4 are in aseptic packaging. In use, firstly, the controller 1 is connected with the handle 2 by the cable component 4, then the adapter 3 is mounted on the handle 2 and the battery pack 16 is mounted on the control module 17, and finally, a proper cartridge is loaded in an outer tube of the adapter 3 to perform the operation of anastomosis. After operation is ended, the control module 17 of the controller 1 is stored according to specified storage requirements, the battery pack 16 and the adapter 3 are subject to waste treatment as disposable components, and the handle 2 and the cable component 4 are sterilized according to operation specifications, and are stored according to storage requirements for use next time. A brand new battery pack 16 is used next time.

### Implementation mode V:

The implementation mode is basically the same as the implementation mode I, and the only difference is that a cable component 4 is not included, the controller 1 can be directly mounted on a handle 2 and is detachably connected with the handle 2, the controller 1 includes a battery pack 16 and a control module 17, and electric energy of the controller 1 is supplied by the battery pack 16, referring to FIG. 6, the adapter 3 and the battery pack 16 are disposable components, and the handle 2 can be reused for many times after being sterilized.

Before use of a reusable powered endoscopic cutter stapler according to implementation mode V, a controller 1, a handle 2 and an adapter 3 are in aseptic packaging. In use, firstly, the controller 1 is directly mounted on one end of the handle 2, then the adapter 3 is mounted on the other end of the handle 2, and finally, a proper cartridge is loaded in an outer tube of the adapter 3 to perform the operation of anastomosis. After operation is ended, the controller and the adapter 3 are subject to waste treatment as disposable components, and the handle 2 is sterilized according to operation specifications, and is stored according to storage requirements for use next time.

According to the reusable powered endoscopic cutter stapler of the present application at least one or multiple components of a controller can be sterilized repeatedly, and can be used for many times, thereby not only greatly lowering the treatment expense of patients, but also greatly reducing the risk of cross infection when instruments are used, improving the use safety and reliability of the instruments, and greatly lightening the overall mass, and a detachable battery pack is used for supplying energy for the controller, so that a doctor can operate more conveniently, without being limited by use occasions.

It should be noted that implementation schemes in the accompanying drawings are merely representative embodiments of the present application, a person skilled in the art may easily understand that the protection scope of the present application is not merely limited in a scope defined by implementation modes in the accompanying drawings, and combination, transformation and variation for implementation modes in the drawings all fall within the protection scope of the present application.

The foregoing disclosed are merely several preferred embodiments of the present application, of course, the protection scope of the present application should be not limited hereby, therefore, equivalent variations made according to claims of the present application still belong to a coverage scope of the present application.

## Claims

1. A reusable powered endoscopic cutter stapler, comprising: a controller, a handle, an adapter and a cable component; the adapter being connected with the handle; one end of the cable component can be detachably connected with the controller, the other end being detachably connected with the handle to be used for transmitting electric energy and command signals; the controller can be reused for many times, and the cable component can be reused for many times after being sterilized.

2. The reusable powered endoscopic cutter stapler according to claim 1, wherein the adapter is detachably connected with the handle; the adapter being a disposable component, and the handle can be reused for many times after being sterilized.

3. The reusable powered endoscopic cutter stapler according to claim 1, wherein the adapter and the handle combine a whole device, the whole device being reused for many times after being sterilized.

4. The reusable powered endoscopic cutter stapler according to claim 1, wherein the adapter and the handle combine a whole device, the whole device being a disposable component.

5. The reusable powered endoscopic cutter stapler according to claim 1, wherein the controller comprises a battery pack and a control module which are detachably connected, electric energy of the controller can be supplied by the battery pack, the battery pack and the adapter being disposable components, and the handle can be reused for many times after being sterilized.

6. The reusable powered endoscopic cutter stapler according to any one of claims 1-5, wherein the controller comprises a battery pack and a control module, the battery pack converting power frequency voltage into DC voltage needed by the handle; the control module comprising a circuit board component and a software program, and being in charge of the mechanism operation and indication of the handle.

7. The reusable powered endoscopic cutter stapler according to any one of claims 1-5, wherein the handle acquires electric energy from the controller, operates the adapter according to the instruction of the controller, including firing or withdrawing a blade, firing blade and indicating the location and direction as well as residual use times of a handle.

8. The reusable powered endoscopic cutter stapler according to any one of claims 1-5, wherein the handle comprises a handle case, a firing handle, a safety trigger, a return button, a stroke indicator light, a residual use time indicator light and a direction indicator light.

9. The reusable powered endoscopic cutter stapler according to any one of claims 1-5, wherein the adapter comprises an outer tube, a rotation collar and a rotation knob; the far end of the outer tube being loaded with a proper cartridge in use to be used for anastomosis.

10. A reusable powered endoscopic cutter stapler, comprising a controller, a handle and an adapter; the adapter being connected with the handle; the controller can be directly mounted on the handle and detachably connected with the handle, the controller comprising a battery pack and a control module, electric energy of the controller can be supplied by the battery pack, the adapter and the battery pack being disposable components, and the handle can be reused for may times after being sterilized.

11. The reusable powered endoscopic cutter stapler according to claim 10, wherein the controller comprises a battery pack and a control module, the battery pack converting power frequency voltage into DC voltage needed by the handle; the control module comprising a circuit board component and a software program, and being in charge of the mechanism operation and indication of the handle.

12. The reusable powered endoscopic cutter stapler according to claim 10, wherein the handle acquires electric energy from the controller and operates and indicates the adapter according to the instruction of the controller, firing blade including firing or withdrawing a blade, and indicating the location and direction as well as residual use times of a handle.

13. The reusable powered endoscopic cutter stapler according to claim 10, wherein the handle comprises a handle case, a firing handle, a safety trigger, a return button, a stroke indicator light, a residual use time indicator light and a direction indicator light.

14. The reusable powered endoscopic cutter stapler according to claim 10, wherein the adapter comprises an outer tube, a rotation collar and a rotation knob; the far end of the outer tube being loaded with a proper cartridge in use to be used for anastomosis.
